# EUROPEAN PATENT SPECIFICATION

(11) **EP 1 677 112 B1**
(45) Date of publication and mention of the grant of the patent: **17.08.2011**
(21) Application number: 04716752.3
(22) Date of filing: 03.03.2004
(51) Int. Cl.: G01N 33/53, G01N 33/531, G01N 33/68

(54) **IMMUNOASSAY**
IMMUNTEST
ESSAI IMMUNOLOGIQUE

(30) Priority: 30.09.2003 JP 2003340412
(43) Date of publication of application: 05.07.2006
(73) Proprietor: MORINAGA & CO., LTD., Tokyo 108-8403 (JP)
(72) Inventor: MURAOKA, Shiroo, Morinaga Inst. Of Biological Sci., Yokohama-shi, Kanagawa 230-0012 (JP); WATANABE, Yumiko, Morinaga Inst. Of Biologic. Sci., Yokohama-shi, Kanagawa 230-0012 (JP); SAKAI, Masatoshi, Morinaga Inst. Of Biologic. Sci., Yokohama-shi, Kanagawa 230-0012 (JP); HONJOH, Tsutomu, Morinaga Inst. Of Biological Sci., Yokohama-shi, Kanagawa 230-0012 (JP)
(74) Representative: Stenbäck, Maria Elisabeth
(86) International application number: PCT/JP2004/002604
(87) International publication number: WO 2005/033704

(56) References cited:
- WO-A-02/04918
- WO-A-89/11100
- JP-A- 9 077 798
- JP-A- 2001 215 228

## Description

### Technical Field

The present invention relates to an immunoassay for highly sensitively measuring a water-sparingly-soluble protein or a protein in a hardly extractable state.

### Background Art

With the rapid advance of molecular biology, demand for higher sensitivity in immunoassay testing is increasing. In the field of life science, evidence is mounting day by day that various kinds of membrane-bound cell surface proteins have a very important role to play in intracellular or intercellular signal transduction. These cell surface proteins are expressed as various subtypes among animals or tissues, and their time-course and spatial expression pattern is strictly regulated. Accordingly, in the life science of today, highly sensitive detection of cell surface proteins is essential for understanding various life phenomena. However, most membrane-bound cell surface proteins are insoluble/sparingly soluble or hardly extractable in an ionic surfactant-free aqueous solvent.

The demand for highly sensitive immunoassays is also increasing in the field of public hygiene. As consumers become more and more concerned about the use of genetically engineered plants, BSE, food allergen and so on, they expect food manufactures to perform highly sensitive detection of these proteins. Particularly, proteins such as food allergens, when present in a processed food, tend to form a complicated complex with other components in the processed food and thus it is experienced that the proteins cannot easily be extracted from the processed food with an ionic surfactant-free aqueous solvent even if the proteins are intrinsically water soluble. For example, proteins tend to bind very strongly to wheatderived gluten in a processed wheat food and are thus hardly extractable in the absence of an ionic surfactant in the aqueous extraction solvent.

Therefore, for realizing a highly sensitive detection of such water-sparingly-soluble/hardly extractable protein by an immunoassay, the specificity and affinity of an antibody used in the assay should be improved to sufficiently detect the presence of a very small amount of such poorly soluble/extractable protein. However, there is a certain limit to the improvement of specificity and affinity.

Accordingly, in such highly sensitive immunoassay, the entire protocol of the assay should be revised in addition to the improvement of the specificity and affinity of the antibody. Particularly, it is considered that the sensitivity of the entire assay can be significantly improved by efficiently extracting the analyte water-sparingly-soluble/hardly extractable protein from a sample, and for this purpose, the solubilization/extraction of the water-sparingly-soluble protein etc. with an ionic surfactant such as sodium dodecyl sulfate (SDS) can be a very powerful technique. However, the ionic surfactant is believed to inhibit antigen-antibody reactions in the subsequent immunoassay so that the antigen-antibody reactions under the presence of the ionic surfactant at high concentration have been avoided.

That is, when relatively high concentration of an ionic surfactant such as SDS is used to efficiently extract a protein and thus extracted protein is measured by the conventional immunoassay, the ionic surfactant should be removed from the extract, or the extract itself should be sufficiently diluted prior to the immunoassay so that the ionic surfactant is reduced to a concentration at which the surfactant is believed not to exert adverse influences on the immunoreaction. Practically, the extract has been pretreated to reduce the concentration of the ionic surfactant therein by a method such as 1) dialysis of the extract with a cellophane tube or the like, 2) exchanging the solvent with a surfactant-free solvent by centrifugal filtration or the like, 3) exchanging the solvent with a surfactant-free solvent by gel filtration or ion-exchange chromatography, or 4) selective precipitation of the surfactant with a chemical substance. However, ionic surfactants readily form micelles with proteins, and therefore selective removal of the surfactants is very difficult in many cases, meaning that the removal treatments as described above could be the cause of significant reduction in the yield of a desired protein. Moreover, the removal treatments are troublesome in the first place. Alternatively, the entire extract may adequately be diluted to reduce the concentration of the ionic surfactant, however the concentration of the extracted protein to be measured is also simultaneously reduced by the dilution, and as a consequence, the detection sensitivity in the subsequent immunoassay is not improved at all. That is, it is generally believed that the use of high concentrations of ionic surfactants for efficiently extracting the water-sparingly-soluble/hardly extractable protein from a sample is incompatible with the subsequent immunoassay in view of the feasibility of the immunoreactions.

Japanese Patent Application Laid-Open Publication No. 9-77798 (JP 9077798A) describes antigen-antibody reactions in the presence of an ionic surfactant SDS (page 5, right column, paragraph 0030; page 6, left column, paragraph 0033; and page 10, left column, paragraph 0051). This reference relates to a stable and crossreaction-free immunoassay of plasma protein CETP (cholesteryl ester transfer protein) having an unstable structure, and in this assay, CETP pre-denatured with 0.001 to 10% (W/V) SDS is detected. In the reference, it is also described that the antigen-antibody reaction between the CETP and the antibody thereagainst can be carried out even in the presence of 0.001 to 0.3% (W/V) SDS, and the particularly preferable range is 0.02 to 0.03% (W/V) SDS. Further, in the Examples therein, CETP pretreated with 0.25% SDS was further diluted 11-fold (by adding 200 µl antibody solution to 20 µl pretreated solution) to adjust the concentration of SDS to about 0.023%. Thereafter, the antigen-antibody reaction was carried out.

That is, in the reference, it is believed that the ionic surfactants such as SDS and the like at a concentration of 0.03% or more inhibit the antigen-antibody reaction in the immunoassay. It is therefore taught by the reference that when the antigen-antibody reaction is carried out, a sample containing high concentration of SDS should be sufficiently diluted such that the concentration of SDS is reduced to 0.03% or less.

WO 02/04918 A describes a method of preparing a sample for an immunoassay wherein complex samples such as bacterial cells are treated with hot detergent. WO 89/11100 A describes the preparation of a specimen for an immunoassay in which the specimen is immersed in a buffered surfactant such as non-ionic, ionic, zwitterionic surfactants and bile salts or derivatives thereof, in order to make more antigen accessible for testing. WO 99/06836 also describes a method of preparing a sample for an immunoassay comprising treating the sample with an anionic surfactant and an amphoteric or non-ionic surfactant or a protein denaturing agent. The reference also describes the use of SDS as the anionic surfactant and the use of urea or thiourea as the protein denaturing agent.

### Disclosure of Invention

Accordingly, the object of the present invention is to provide a method wherein a protein sparingly soluble in water or a protein in a hardly extractable state, which is required to be detected with very high sensitivity, is detected sensitively and easily in subsequent immunoreaction while high efficiency of extraction from a sample is maintained.

As opposed to the common technical knowledge in the prior art, it was surprisingly found that even in the presence of an ionic surfactant at high concentration, the antigen-antibody reaction itself is not inhibited to such a level as to be undetectable. It was also found that even for a certain protein whose antigen-antibody reaction is apparently inhibited by a high concentration of ionic surfactant, the antigen-antibody reaction can be satisfactorily effected by using an antibody raised against the protein which was previously denatured with the ionic surfactant. On the basis of these findings, the present inventors completed a highly sensitive and easy immunoassay of a protein sparingly soluble in water or a protein in a hardly extractable state. According to the present invention, therefore, there is provided a highly sensitive and easy immunoassay characterized in that a protein sparingly soluble in water or a protein in a hardly extractable state, which is contained in a sample, can be extracted/solubilized with an aqueous solvent containing an ionic surfactant at relatively high concentration, and then the protein in the resulting extract can be detected directly by an immunoassay without exchanging the solvent of the extract with another solvent, or without substantially diluting the extract such that the concentration of the protein is not reduced to an undetectable level.

Accordingly, the present invention provides an immunoassay for detecting the presence of a water-sparingly-soluble/hardly extractable protein in a sample, comprising the steps of:
(I) extracting and/or solubilizing a water-sparingly-soluble/hardly extractable protein in a sample with an aqueous solvent containing an ionic surfactant,
(II) adding an antibody obtained by using the water-sparingly-soluble/hardly extractable protein as immunogen previously denatured with the ionic surfactant used in step (I) to:
   a) the protein solution obtained in the step (I) above without substantially diluting the solution, or
   b) a dilution wherein the protein solution obtained in the step (I) above is diluted in such a range that the concentration of the ionic surfactant is not reduced to 0.03% (W/V) or less,
   whereby an antigen-antibody complex between the water-sparingly-soluble/hardly extractable protein and the antibody is formed, and
(III) detecting the formed antigen-antibody complex.

It was found that even if the concentration of the ionic surfactant in the aqueous solvent in the step (I) is for example higher than 0.3% (W/V), formation of an antigen-antibody complex in the sample (protein) solution extracted with said aqueous solvent is not inhibited. That is, the antigen-antibody complex in the step (II) can be formed in the presence of the ionic surfactant at a concentration of higher than 0.3% (W/V), preferably 1% (W/V) or more. Even if the sample solution should be diluted for the purpose of quantitative analysis etc., it is not necessary that the ionic surfactant is diluted to a concentration of 0.03% (W/V) or less believed in the prior art to be necessary for excellent antigen-antibody reaction, and this means that the method of the present invention can be practiced without sacrificing the high extraction power of the ionic surfactant.

The ionic surfactant in the present invention can be selected from sodium dodecyl sulfate, lithium dodecyl sulfate, sodium lauryl sarcosine, hexadecyltrimethyl ammonium bromide, hexadecyltrimethyl ammonium chloride, hexadecyl pyridinium chloride etc., and if necessary these surfactants may be mixed. In particular, sodium dodecyl sulfate (SDS) used frequently in the field of biochemistry can be mentioned as a preferable example because of its availability etc.

It was revealed that the aqueous solvent in the step (I) can also contain a reducing agent such as 2-mercaptoethanol, dithiothreitol etc. which can further denature the protein. Accordingly, non-limiting examples of the preferable aqueous solvent in the step (I) include an aqueous solvent containing 1% (W/V) sodium dodecyl sulfate and 1M 2-mercaptoethanol.

For securing measurement stability and reproducibility, the protein solution is preferably further boiled in the step (I), and the boiling is continued preferably at 80°C or more for 5 minutes or more.

The method of the present invention is particularly advantageous in highly sensitive measurement of ovalbumin, ovomucoid, casein, β-lactoglobulin, buckwheat protein, wheat protein and peanut protein which are in a hardly extractable state. Ovalbumin, ovomucoid, casein etc. are easily soluble in water in themselves, but the proteins when present in complicated matrix in processed food etc. may be hardly extracted with a usual aqueous solvent. The method of the present invention can be used very effectively in highly sensitive measurement of the protein in such a hardly extractable state.

Thus, the method of the present invention, owing to the excellent effect of an ionic surfactant on solubilization of protein and the new finding of the nature of the antigen-antibody reaction in the presence of an ionic surfactant at high concentration, can be utilized very effectively in life science studies of today and in food quality assurance where highly sensitive detection of a protein sparingly soluble in water, or a protein in a hardly extractable state, is required.

### Brief Description of Drawings

Fig. 1 shows results of measurement of standard ovomucoid (absorbance at a measurement wavelength of 450 nm and a side wavelength of 630 nm) by the illustrative method of the present invention (solid phase sandwich method based on the principle of EIA) after serial dilution of the standard.
Fig. 2 shows results of measurement of ovomucoid-containing samples of unknown concentration (Samples a to c) (absorbance at a measurement wavelength of 450 nm and a side wavelength of 630 nm) in the measurement system by the illustrative method of the present invention (solid phase sandwich method based on the principle of EIA).
Fig. 3 shows results of measurement (Reference Example) of ovalbumin in the presence of HDTMAB, HDTMAC and HDPC. The concentration (%) of each surfactant is shown on the abscissa and absorbance on the ordinate.
Fig. 4 shows results of measurement (Reference Example) of ovalbumin in the presence of LDS, sodium lauryl sarcosine and SDS. The concentration (%) of each surfactant is shown on the abscissa and absorbance on the ordinate.
Fig. 5 shows results of measurement of ovomucoid in the presence of HDTMAB, HDTMAC and HDPC. The concentration (%) of each surfactant is shown on the abscissa and absorbance on the ordinate.
Fig. 6 shows results of measurement of ovomucoid in the presence of LDS, sodium lauryl sarcosine and SDS. The concentration (%) of each surfactant is shown on the abscissa and absorbance on the ordinate.
Fig. 7 shows results of measurement (Reference Example) of ovalbumin in the presence of Tween 20, Luburol PX, Triton X-100 and DOC. The concentration (%) of each surfactant is shown on the abscissa and absorbance on the ordinate.
Fig. 8 shows results of measurement (Reference Example) of ovomucoid in the presence of Tween 20, Luburol PX, Triton X-100 and DOC. The concentration (%) of each surfactant is shown on the abscissa and absorbance on the ordinate.
Fig. 9 shows results of measurement of casein in the presence of SDS. The concentration (%) of the surfactant (SDS) is shown on the abscissa and absorbance on the ordinate. (closed square) is in the presence of antigen, and (closed diamond) is in the absence of antigen (control).
Fig. 10 shows results of measurement of β-lactoglobulin in the presence of SDS. The concentration (%) of the surfactant (SDS) is shown on the abscissa and absorbance on the ordinate. (closed square) is in the presence of antigen, and (closed diamond) is in the absence of antigen (control).
Fig. 11 shows results of measurement of buckwheat protein in the presence of SDS. The concentration (%) of the surfactant (SDS) is shown on the abscissa and absorbance on the ordinate. (closed square) is in the presence of antigen, and (closed diamond) is in the absence of antigen (control).
Fig. 12 shows results of measurement of wheat protein (gliadin) in the presence of SDS. The concentration (%) of the surfactant (SDS) is shown on the abscissa and absorbance on the ordinate. (closed square) is in the presence of antigen, and (closed diamond) is in the absence of antigen (control).
Fig. 13 shows results of measurement of peanut protein in the presence of SDS. The concentration (%) of the surfactant (SDS) is shown on the abscissa and absorbance on the ordinate. (closed square) is in the presence of antigen, and (closed diamond) is in the absence of antigen (control).
Fig. 14 shows results of measurement of ovalbumin by using the anti-ionic surfactant-denatured protein antibody according to the present invention.
Fig. 15 shows the effect of boiling of an ovalbumin sample solution on the stability of detection sensitivity with time in the method of using the anti-ionic surfactant-denatured protein antibody according to the present invention. The Reference Example shows results of measurement where an antibody to the native protein was used, and the sample solution was heated or not heated.
Fig. 16 shows the effect of boiling of an ovomucoid sample solution on the stability of detection sensitivity with time in the method of using the anti-ionic surfactant-denatured protein antibody according to the present invention. The reference example shows results of measurement where an antibody to the native protein was used, and the sample solution was heated or not heated.
Fig. 17 shows the effect of boiling of a peanut protein sample solution on the stability of detection sensitivity with time in the method of using the anti-ionic surfactant-denatured protein antibody according to the present invention. The reference example shows results of measurement where an antibody to the native protein was used, and the sample solution was heated or not heated.
Fig. 18 shows the effect of boiling of a buckwheat protein sample solution on the stability of detection sensitivity with time in the method of using the anti-ionic surfactant-denatured protein antibody according to the present invention. The reference example shows results of measurement where an antibody to the native protein was used, and the sample solution was heated or not heated.

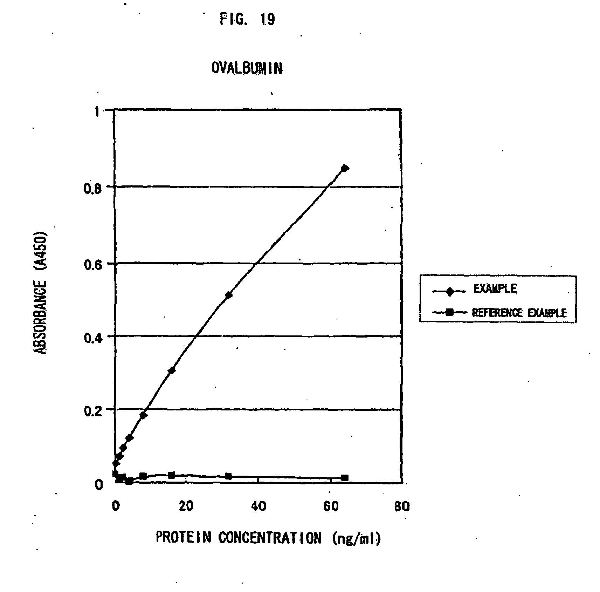

Fig. 19 shows assay sensitivity in a preferable aspect of the present invention where use of the anti-ionic surfactant-denatured protein antibody was combined with boiling of the sample solution. The example shows results of measurement where ovalbumin at each concentration was dissolved in a buffer solution (PBS, pH 6.5) containing 1% SDS and 1M 2-mercaptoethanol to prepare a sample solution, and the sample solution was heated for 5 minutes in a hot water bath at 100°C, then cooled and measured by ELISA, while the reference example shows results of measurement where an antibody to native ovalbumin was used, and the sample solution was heated.

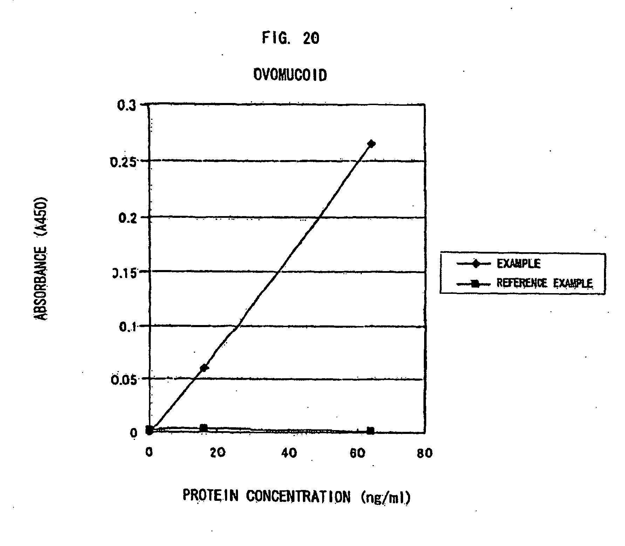

Fig. 20 shows assay sensitivity in a preferable aspect of the present invention where use of the anti-ionic surfactant-denatured protein antibody was combined with boiling of the sample solution. The example shows results of measurement where ovomucoid at each concentration was dissolved in a buffer solution (PBS, pH 6.5) containing 1% SDS and 1M 2-mercaptoethanol to prepare a sample solution, and the sample solution was heated for 5 minutes in a hot water bath at 100°C, then cooled and measured by ELISA, while the reference example shows results of measurement where an antibody to native ovomucoid was used, and the sample solution was heated.

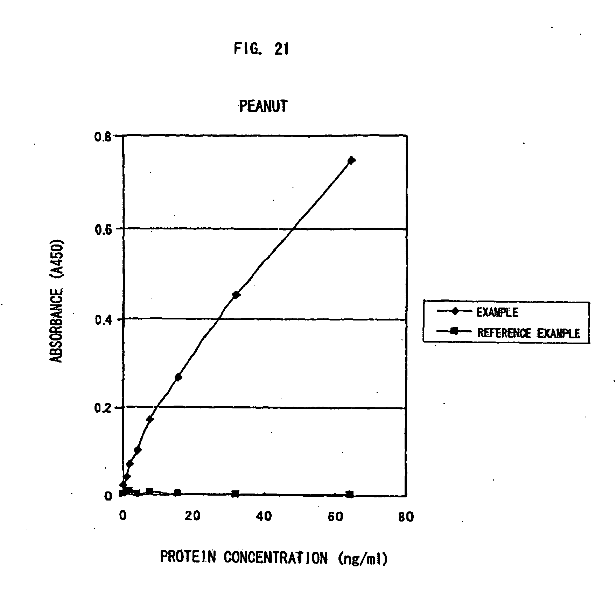

Fig. 21 shows assay sensitivity in a preferable aspect of the present invention where use of the anti-ionic surfactant-denatured protein antibody was combined with boiling of the sample solution. The example shows results of measurement where peanut protein at each concentration was dissolved in a buffer solution (PBS, pH 6.5) containing 1% SDS and 1M 2-mercaptoethanol to prepare a sample solution, and the sample solution was heated for 5 minutes in a hot water bath at 100°C, then cooled and measured by ELISA, while the reference example shows results of measurement where an antibody to native peanut protein was used, and the sample solution was heated.

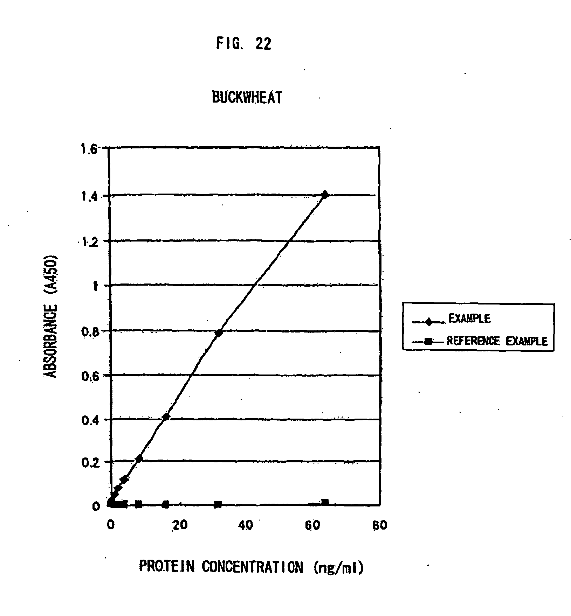

Fig. 22 shows assay sensitivity in a preferable aspect of the present invention where use of the anti-ionic surfactant-denatured protein antibody was combined with boiling of the sample solution. The example shows results of measurement where buckwheat protein at each concentration was dissolved in a buffer solution (PBS, pH 6.5) containing 1% SDS and 1M 2-mercaptoethanol to prepare a sample solution, and the sample solution was heated for 5 minutes in a hot water bath at 100°C, then cooled and measured by ELISA, while the reference example shows results of measurement where an antibody to native buckwheat protein was used, and the sample solution was heated.

### Best Mode for Carrying Out the Invention

The "water-sparingly-soluble/hardly extractable protein" as the subject of the present invention is a protein which does not show substantial solubility in ionic surfactant-free pure water or a generally used physiological buffer solution or cannot be substantially extracted from a sample with such a buffer solution, but with a buffer solution or the like containing the ionic surfactant in the present invention, the protein can be solubilized or extracted to a concentration at which it can be detected by subsequent immunoassays. In other words, the "water-sparingly-soluble/hardly extractable protein" in the present invention shows significantly higher solubility and/or extraction efficiency with the ionic surfactant-containing buffer solution or the like than with ionic surfactant-free pure water or a buffer solution. Typically, the "water-sparingly-soluble/hardly extractable protein" as the subject of the present invention can show improved solubility and/or extraction efficiency in the ionic surfactant-containing buffer solution of the present invention, which is at least 5 times, preferably at least 10 times, more preferably at least 50 times as high as the solubility and extraction efficiency in ionic surfactant-free pure water or a buffer solution. Non-limiting examples of the water-sparingly-soluble/hardly extractable protein include structural proteins and membrane-bound cell surface proteins. In particular, membrane proteins responsible for biochemically important functions, for example cell surface receptors and cell-adherent factors, are interesting. Other examples of the water-sparingly-soluble/hardly extractable protein include food allergen proteins present in processed food etc. For example, ovalbumin, ovomucoid, casein, β-lactoglobulin, buckwheat protein, wheat protein and peanut protein are considered important as food allergen proteins. These proteins include those soluble in water in themselves, but may be in a hardly extractable state when complexed strongly with other components in processed food and integrated in the matrix of the food. The water-soluble proteins in such a hardly extractable state also fall under the scope of the water-sparingly-soluble/hardly extractable protein referred to herein insofar as they can be extracted for the first time by the effect of the ionic surfactant in the present invention.

The "sample" referred to in the present invention can be a fluid, semi-solid or solid, or a mixture thereof, suspected to contain the water-sparingly-soluble/hardly extractable protein, and can be advantageously a sample including a solid matrix having the water-sparingly-soluble/hardly extractable protein integrated therein, which includes, but is not limited to, not only cells, cell membranes, tissues and organs but also foods and materials.

In the method of the present invention, the water-sparingly-soluble/hardly extractable protein described above is solubilized/extracted with the ionic surfactant-containing "aqueous solvent". The "aqueous solvent" means water; solutions of salts such as sodium chloride, potassium chloride and sodium bicarbonate; various buffer solutions used generally in the field of biochemistry, for example a phosphate buffer, Tris-HCl buffer and citrate buffer; and an alkali or acidic solution of which pH was adjusted with sodium hydroxide, hydrochloric acid etc. The aqueous solvent can also contain auxiliary components for further improving the solubility and extraction efficiency of the protein. For example, a chelating compound such as ethylenediaminetetraacetic acid (EDTA), an enzyme such as phospholipase, and a nonionic surfactant for controlling HLB can be added to the aqueous solvent. A protease inhibitor for controlling degradation of the protein in a solution during extraction or storage, an antimicrobial agent such as sodium azide for preventing propagation of microorganisms, and an antioxidant such as ascorbic acid may also be added. In addition, polar organic solvents such as glycerol and ethanol can also be added to the aqueous solvent in such a range that the subsequent immunoassay is feasible.

The ionic surfactant of the invention added to the aqueous solvent may be any known surfactant insofar as it can substantially improve the solubilization and extraction of the water-sparingly-soluble/hardly extractable protein. Preferably, the ionic surfactant is selected from the group consisting of sodium dodecyl sulfate, lithium dodecyl sulfate, sodium lauryl sarcosine, hexadecyltrimethyl ammonium bromide, hexadecyltrimethyl ammonium chloride, hexadecyl pyridinium chloride and a mixture thereof. From the viewpoint of availability and handling, sodium dodecyl sulfate (SDS) can be mentioned as a particularly preferable ionic surfactant. In particular, SDS is an ionic surfactant widely used in electrophoresis such as SDS-PAGE, and is a preferable ionic surfactant for the purpose of facilitating correlation of the electrophoresis with the immunoassay.

The concentration of the ionic surfactant added may be any concentration at which the solubilization and extraction of the water-sparingly-soluble/hardly extractable protein as the object of this invention can be substantially achieved, but usually the ionic surfactant is added at a concentration of 0.1% (W/V) or more, preferably 0.3% (W/V) or more, or even 0.5% (W/V) or more. The ionic surfactant is added more preferably at a concentration of 1% (W/V) or more to the aqueous solvent, and the ionic surfactant at a high concentration of about 10% (W/V) can also be used. In the method of the present invention, the protein solubilized/extracted with the aqueous solvent containing the ionic surfactant at such high concentration can be detected by subsequent immunoassay while the concentration of the ionic surfactant is substantially maintained.

In addition to the high concentration of ionic surfactant, a reducing agent, typically 2-mercaptoethanol, dithiothreitol (DTT), sodium cyanoborohydride (SCBH), dimethyl amine borane (DMAB), sodium borohydride (SBH) or cysteine, is preferably added to the particularly preferable aqueous solvent in the present invention. This is because it is often experienced that the reproducibility of subsequent immunoassay can be improved by adding the reducing agent, although the principle of this improvement is not revealed. Preferably, the reducing agent may be added at a concentration of about 1mM to 2M, usually at a concentration of about 1M, to the aqueous solvent.

As described above, the particularly preferable ionic surfactant-containing aqueous solvent of the invention can be for example a phosphate buffer containing 1% (W/V) sodium dodecyl sulfate and 1M 2-mercaptoethanol.

As is already evident, the advantage of the present invention lies in that the water-sparingly-soluble/hardly extractable protein is solubilized/extracted with the ionic surfactant having a strong ability to solubilize the protein, and the resulting extract solution can be subjected to the subsequent immunoassay without substantial diluting the extract. According to the conventional technical common sense, it has been considered that the desired antigen-antibody reaction cannot be achieved when an ionic surfactant such as SDS is present at relatively high concentration (for example 0.03% or more) in a reaction solution. In the typical conventional immunoassay directed to the water-sparingly-soluble/hardly extractable protein, therefore, troublesome pretreatment or significant dilution of a sample solution containing an ionic surfactant at high concentration is required prior to the addition of an antibody to the sample solution; that is, by this pretreatment or dilution, the concentration of the ionic surfactant in the sample solution must be reduced to about 0.03%, prior to the antigen-antibody reaction. Accordingly, a sample solution containing a sparingly soluble protein extracted with 10% (W/V) SDS, for example, should be diluted at least 300-fold (0.03% SDS) prior to the antigen-antibody reaction, which means that the detection sensitivity of the protein is reduced to 1/300.

In the method of the present invention, on the other hand, the antigen-antibody reaction can be satisfactorily effected even in the presence of the ionic surfactant at high concentration, and the specificity and affinity of the antigen-antibody reaction can be significantly improved by use of an antibody to a denatured protein obtained by denaturing the objective protein with the same ionic surfactant, and on the basis of this finding, an improvement in the sensitivity of the whole of the assay is contemplated by using a solution of a protein solubilized with an aqueous solvent containing e.g. 1% SDS as a sample solution in the subsequent antigen-antibody reaction without substantial diluting it. In the method of the present invention, a sparingly soluble protein solubilized with a solvent containing at least 10% (W/V) SDS can be measured in the 10% SDS solution by the antigen-antibody reaction. For accurate quantification of an antigen protein in a sample solution, the suitable serial dilution of the solution may often be required for the purpose of confirming the linearity of measurements and etc. In the present invention, it is not necessary to reduce the concentration of SDS in a sample solution even in such serial dilution as above, thus avoiding the possible precipitation of the water-sparingly-soluble protein as a result of the reduction in the concentration of SDS. In the present invention, it is therefore not necessary that the concentration of the ionic surfactant is reduced to 0.03% (W/V) or less even if a sample solution containing the ionic surfactant at high concentration is to be diluted depending on the case.

As described above, one of the important features of the present invention is that the present immunoassay based on antigen-antibody reaction is performed by using a specific antibody to a water-sparingly-soluble/hardly extractable protein previously denatured with a specific ionic surfactant wherein the specific antibody to the denatured protein is obtained from the immunized animal administered with the denatured protein as the immunogen.

Specifically, when it is known that a certain water-sparingly-soluble/hardly extractable protein in a sample can be extracted with an aqueous solvent containing SDS, the protein is denatured with SDS, and the denatured protein is administered as immunogen into an animal to be immunized in preparing the antibody of the present invention. The denaturation of the protein can be easily achieved by dissolving or suspending the analyte water-sparingly-soluble/hardly extractable protein in a solution containing an ionic surfactant at high concentration and then leaving it at room temperature at least overnight. In a preferable example of the present invention wherein the aqueous solvent for solubilization/extraction of the protein contains an additional reducing agent such as 2-mercaptoethanol together with SDS, the denaturation of the immunogen protein may preferably be conducted in the presence of 2-mercaptoethanol and SDS. In such a case, the analyte water-sparingly-soluble/hardly extractable protein can be suspended/dissolved in an aqueous solvent containing 1% SDS and 1M 2-mercaptoethanol, then left at room temperature at least overnight and can be used as the denatured protein immunogen used in preparing the antibody of the present invention.

In administering thus obtained denatured protein into an animal to be immunized, any protocol known by those skilled in the art can be used, and the optimization of the protocol is also easy for those skilled in the art. As the animal immunized, a mouse, rat, sheep, rabbit or the like may be used.

In particular, when the antibody of the present invention is used in the form of a polyclonal antibody, the polyclonal antibody is prepared from an antiserum of an immunized animal. Specifically, an antiserum from an immunized animal can be obtained for example by subcutaneously injecting the immunogen containing an adjuvant into an animal to be immunized, repeating this subcutaneous administration at suitable intervals (for example 1 week) predetermined times (for example 5 times), collecting the whole blood after the final immunization, and separating the antiserum. Such methods are described in for example Current Protocols in Immunology, Chap. 2.4, published by John Wiley & Sons, Inc., New York. Purification of the polyclonal antibody from the antiserum can be achieved by covalently immobilizing the denatured protein used in immunization of the animal, onto resin for chromatography, for example, CNBr-activated Sepharose or HiTrap® NHS-activated (manufactured by Amersham Pharmacia), then applying the antiserum onto the immobilization resin to specifically adsorb the antibody in the antiserum onto the resin, and recovering the antibody adsorbed on the resin, by elution with a suitable buffer or chaotropic ions. The polyclonal antibody may also be purified by any other methods.

When the antibody of the present invention is obtained as a monoclonal antibody, a spleen cell of an immunized mouse is fused with a parent cell for cell fusion, such as myeloma cell strain, by techniques known by those skilled in the art, and a suitable fusion cell is selected from the resulting hybridomas, then cloned, and cultured in vitro or in vivo, and a highly specific monoclonal antibody is collected from the culture mixture.

As the antibody of the present invention, use can be made of not only the polyclonal/monoclonal antibodies described above, but also reactive antibody fragments obtained by enzymatically digesting the antibodies. Examples of the antibody fragments include an Fab fragment, an Fab' fragment, an F(ab')₂ fragment, an F(v) fragment, an H-chain monomer, an L-chain monomer or dimer, and a dimer consisting of one H chain and one L chain. The fragment can be obtained by digesting the complete antibody with a protease such as pepsin or papain, which may be followed by treatment if necessary with a reducing agent. The H- or L-chain monomer can also be obtained by treating the complete antibody with a reducing agent such as dithiothreitol and then separating the purified chain.

In the present invention, an antibody to the ionic surfactant-denatured protein is preferably used in measurement, but it was revealed that for a certain protein, an antibody to its denatured protein is not always necessary, and an antibody to its native protein may also be used to achieve allowable detection. However, it was observed that in detection by using an antibody to such a native protein, the detection sensitivity is generally decreased in proportion to a period during which an ionic surfactant-containing sample solution is left. That is, when a specific water-sparingly-soluble/hardly extractable protein is extracted with an ionic surfactant-containing aqueous solvent and then subjected relatively immediately to subsequent antigen-antibody reaction, formation of a relatively stable antigen-antibody complex is recognized, but after the extraction, the rate of formation of the complex is reduced with time. This means that in an immunoassay using an antibody to the native protein, the reproducibility of the assay can be deteriorated in time-dependent manner before the assay is completed.

On the other hand, it was found that such change in the detection with time is negligible when the antibody to the denatured protein in the present invention is used. It was also found that, particularly when the extract is previously boiled, highly reproducible assay can be carried out. That is, in a further preferable aspect of the present invention, a water-sparingly-soluble/hardly extractable protein is extracted with an aqueous solvent containing an ionic surfactant at high concentration, and then the resulting protein solution is boiled, e.g., heated at a temperature of 80°C or more for 5 minutes or more and then cooled, and an antibody to the ionic surfactant-denatured protein is added to the solution to form an antigen-antibody complex.

Thus, there can be provided a highly sensitive immunoassay protocol capable of effectively achieving the antigen-antibody reaction after efficiently solubilizing/extracting the water-sparingly-soluble/hardly extractable protein and without sacrificing a high degree of extraction by dilution etc.

The immunoassay of the present invention includes detection of the antigen-antibody complex formed in the manner described above, and it would be understood that this detection can be carried out by any methods known by those skilled in the art. For example, in sandwich immunoassay as one example of the immunoassay of the present invention, the antibody to the ionic surfactant-denatured protein in the present invention can be used for coating a solid-phase such as the well bottom to provide a capture-side antibody, while another antibody (which may be the same or different from the capture-side antibody) to the denatured protein can be labeled with a radioactive substance, a colored particle or an enzyme to provide a detection-side antibody. After the ionic surfactant-containing sample solution is added to a well having the capture-side antibody and incubated for a predetermined time, the sample extract is removed from each well, then the well is sufficiently washed with a suitable buffer solution, and the detection-side antibody is added to the well. After incubation for a predetermined time, the well is washed, and then formation of a capture-side antibody/analyte/detection-side antibody complex is detected. This detection depends on the properties of the labeling substance with which the detection-side antibody was labeled. The amount of radiation is detected when the label is a radioactive substance, or coloring intensity or absorbance is detected when the label is a colored particle, or absorbance after addition of a suitable substrate to the well and predetermined incubation is detected when the label is an enzyme (ELISA method). The enzyme used in enzyme labeling in the ELISA method is not particularly limited, and an enzyme such as horseradish peroxidase or alkaline phosphatase can be advantageously used. In the case of labeling with horseradish peroxidase, 3,3',5,5'-tetramethylbenzidine or the like can be used as the substrate for the enzyme. When alkaline phosphatase is used, p-nitrophenylphosphoric acid is mentioned as the substrate. On the basis of the measurement result of the immunoassay, the water-sparingly-soluble/hardly extractable protein as the subject of the present invention can be detected.

Those skilled in the art given the above description can sufficiently utilize the present invention without any further description. Hereinafter, the Examples are given only for explanation. Hereinafter, concentration (%) is expressed in weight/volume (W/V) % unless otherwise specified.

### Example 1

### Measurement of ovomucoid in a SDS-containing sample by the solid phase sandwich method (use of an antibody to native ovomucoid)

### (1) Immobilization of an anti-ovomucoid antibody

A rabbit anti-(native) ovomucoid polyclonal antibody was dissolved in an amount of 1 µg/ml in a carbonate buffer (pH 9.6). This solution was pipetted in a volume of 100 µl/well onto a microtiter plate (micromodule plate Maxsorp-F8 manufactured by Nunc) and left at ambient temperature for 2 hours.

### (2) Blocking of the microtiter plate

After the antibody solution was removed from each well, 300 µl blocking solution (20 mM Tris-HCl buffer (pH 7.4) containing 150 mM NaCl, 0.05% Tween 20, 0.1% bovine serum albumin) was added to each well and left at ambient temperature for 2 hours.

### (3) Measurement of ovomucoid

After the blocking solution was removed from each well, 100 µl solution of an ovomucoid standard (trade name: Egg trypsin inhibitor, manufactured by Nacalai Tesque) diluted serially with a sample diluent (20 mM Tris-HCl buffer (pH 7.4) containing 0.1% bovine serum albumin, 150 mM NaCl, 0.05% Tween 20, and 0%, 0.01%, 0.05%, 0.1%, 0.5% or 1% SDS) was added to each well and left at ambient temperature for 2 hours. (In the measurement of various samples shown below, when the amount of ovomucoid contained in a sample was outside of the measurement range of the above system, the sample was further diluted before measurement with a sample diluent such that the amount of ovomucoid was within the measurement range of the system.)

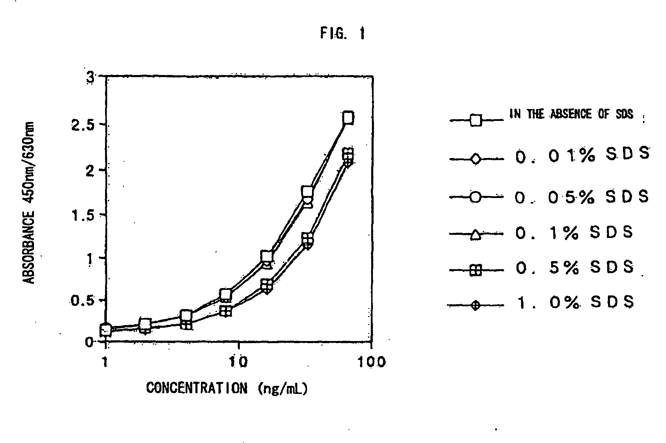

Then, each well was washed 6 times with 300 µl washing solution, and 100 µl solution of a horseradish peroxidase-labeled anti-ovomucoid polyclonal antibody previously diluted with 20 mM Tris-HCl buffer (pH 7.4) containing 0.1% BSA, 150 mM NaCl and 0.05% Tween was added to each well and left at ambient temperature for 30 minutes. Then, each well was washed with 5 times with 300 µl washing solution, and a solution of TMB (3,3',5,5'-tetramethylbenzidine) was added in a volume of 100 µl/well, and then reacted in the dark at ambient temperature for 10 minutes. Thereafter, 100 µl of 1N sulfuric acid was added to each well to terminate the reaction. The absorbance of each well was measured at a major wavelength of 450 nm and a side wavelength of 630 nm with a microtiter plate reader. The resulting standard carve is shown in Fig. 1.

As can be seen from Fig. 1, it was confirmed that in this measurement system, ovomucoid can be quantified even in the presence of SDS because the standard curve of the sample in 0.01 to 1.0% SDS solutions was almost the same as the curve of the sample in SDS-free solutions.

When the amounts of ovomucoid in various samples were actually measured, the amounts of ovomucoid in the samples were determined on the basis of the standard curve obtained in this manner.

### (4) Immunoassays of samples

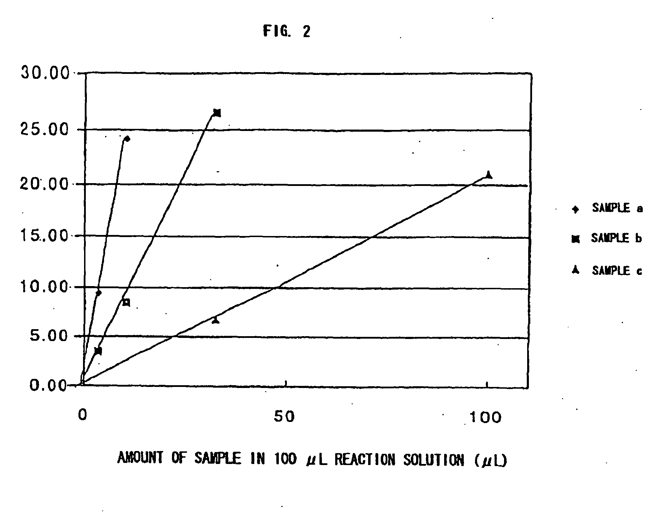

According to the test method described above, immunoassays of samples were actually conducted. Sample solutions from three kinds of commercially available mayonnaise products were prepared by 3-fold serial dilutions of the mayonnaise samples with a sample diluent containing 1% SDS, and on the basis of a standard curve prepared from simultaneously measured standard ovomucoid, the ovomucoid concentrations of the samples were determined. As shown in the results in Fig. 2, excellent linearity passing through the origin of the coordinates was obtained.

### (5) Recovery test

Antigen recovery tests were conducted according to the test method as described above.

Three kinds of commercially available biscuit products were used as samples to prepare extracts containing 1% SDS. That is, each biscuit was uniformly powdered, and 2 g of the powder was weighed and collected. 38 ml sample diluent containing 1% SDS was added to the powder and homogenized twice for 30 seconds with a homogenizer, and the resulting mixture was centrifuged at 3,000 x g for 20 minutes. The resulting supernatant was filtered with a 5A filter paper, and the filtrate was used as a food extract (extract containing 1% SDS) in measurement. Then, standard ovomucoid was added to the extract and subjected to immunoassay, and the recovery of the added ovomucoid was determined from the measurements. The recovery is expressed as the ratio of (amount of the antigen detected in the sample further added the antigen - amount of the antigen detected in the original sample)/(amount of the added antigen [%]).

As shown in Table 1, the recovery was as high as 88.7 to 96.1%, indicating that ovomucoid in the food can be measured accurately and sharply by the method of the present invention.

**Table 1. Results of Recovery Test**

| | Concentration of the antigen originally contained in the sample (ng / mL) | Concentration of the added antigen (ng / mL) | Measurements after addition (ng / mL) | Recovery (%) |
|---|---|---|---|---|
| Sample 1 | 5.14 | 11.32 | 15.18 | 88.69 |
| | 5.14 | 22.79 | 25.54 | 89.51 |
| Sample 2 | 8.44 | 11.32 | 19.06 | 93.80 |
| | 8.44 | 22.79 | 29.87 | 94.02 |
| Sample 3 | 17.98 | 11.32 | 28.86 | 96.11 |
| | 17.98 | 22.79 | 39.30 | 93.55 |

### Example 2

### Measurement of ovomucoid and ovalbumin in samples containing various ionic surfactants or nonionic surfactants by the solid phase sandwich method (use of an anti-native-protein antibody)

The influence of SDS, lithium dodecyl sulfate (LDS) and sodium lauryl sarcosine as the anionic surfactant in the present invention, hexadecyltrimethyl ammonium bromide (HDTMAB), hexadecyltrimethyl ammonium chloride (HDTMAC) and hexadecyl pyridinium chloride (HDPC) as the cationic surfactant, Tween 20, Luburol PX and Triton X-100 as the nonionic surfactant for reference, and deoxycholic acid (DOC) as a surfactant having a steroid skeleton, on the immunoassay system of ovomucoid and ovalbumin (Reference Example) was examined under the same conditions as in Example 1.

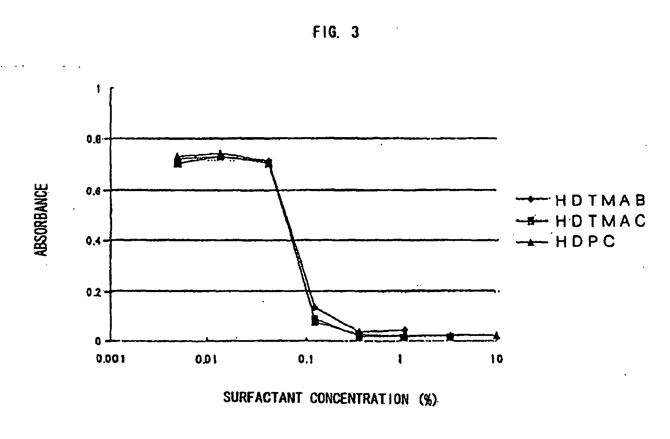

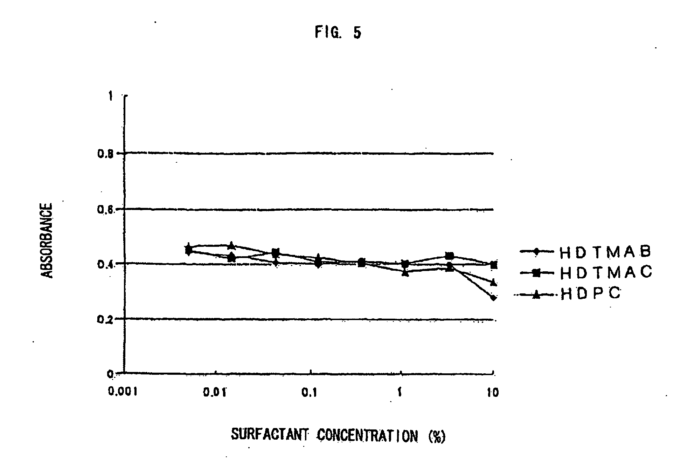

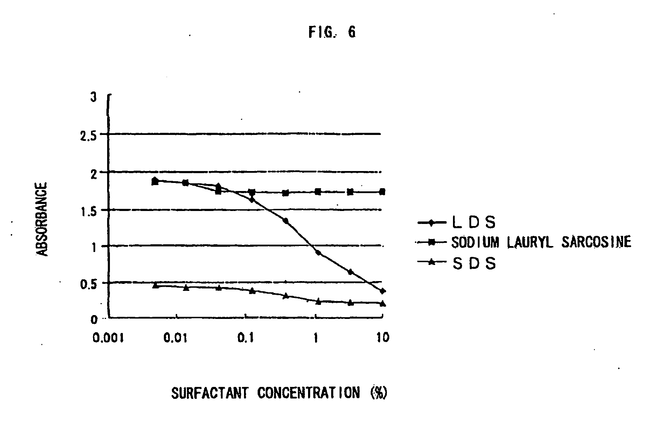

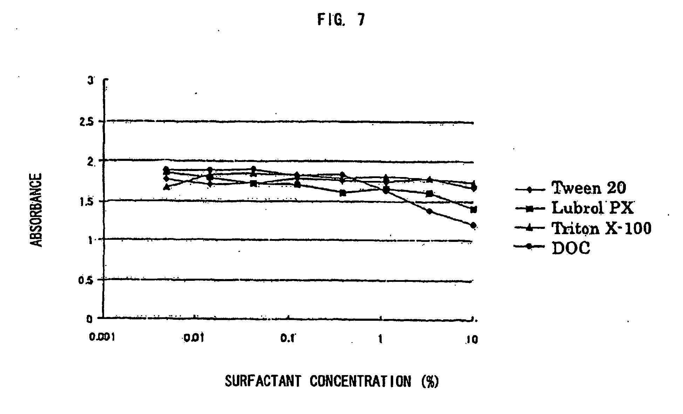

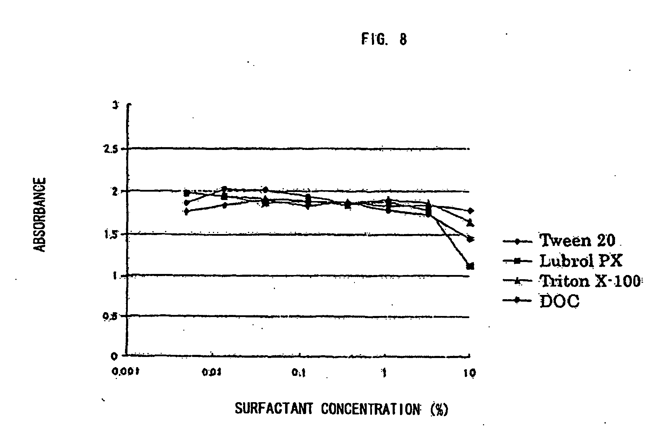

That is, samples containing 64 ng/ml ovalbumin and 64 ng/ml ovomucoid were measured in immunoassays in the presence of each of the above surfactants at various concentrations, and the absorbance was determined. The results are shown in Tables 2 to 5 and Figs. 3 to 8.

**Table 2. Influence of Ionic Surfactants in the Ovalbumin Measurement System**

| Surfactant concentration (%) | Anionic surfactant | | | Cationic surfactant | | |
|---|---|---|---|---|---|---|
| | LDS | Lauroylsarcosine Na | SDS | HDTMAB | HDTMAC | HDPC |
| 0.005 | 1.743 | 1.805 | 0.751 | 0.725 | 0.707 | 0.735 |
| 0.014 | 1.782 | 1.720 | 0.746 | 0.733 | 0.736 | 0.748 |
| 0.041 | 1.260 | 1.713 | 0.600 | 0.715 | 0.707 | 0.718 |
| 0.123 | 0.005 | 1.570 | 0.033 | 0.134 | 0.089 | 0.076 |
| 0.370 | 0.004 | 1.360 | 0.024 | 0.034 | 0.016 | 0.028 |
| 1.110 | 0.004 | 1.185 | 0.018 | 0.045 | 0.016 | 0.023 |
| 3.330 | 0.002 | 1.055 | 0.015 | N. D | 0.017 | 0.023 |
| 10.000 | 0.002 | 0.886 | 0.021 | N. D | N. D | 0.023 |

**Table 3. Influence of Ionic Surfactants in the Ovomucoid Measurement System**

| Surfactant concentration (%) | Anionic surfactant | | | Cationic surfactant | | |
|---|---|---|---|---|---|---|
| | LDS | Lauroylsarcosine Na | SDS | HDTMAB | HDTMAC | HDPC |
| 0.005 | 1.898 | 1.870 | 0.459 | 0.444 | 0.456 | 0.465 |
| 0.014 | 1.849 | 1.857 | 0.439 | 0.432 | 0.419 | 0.469 |
| 0.041 | 1.808 | 1.738 | 0.433 | 0.410 | 0.444 | 0.439 |
| 0.123 | 1.639 | 1.729 | 0.384 | 0.402 | 0.408 | 0.424 |
| 0.370 | 1.336 | 1.719 | 0.319 | 0.412 | 0.410 | 0.406 |
| 1.110 | 0.904 | 1.731 | 0.239 | 0.398 | 0.405 | 0.376 |
| 3.330 | 0.644 | 1.732 | 0.221 | 0.402 | 0.434 | 0.386 |
| 10.000 | 0.381 | 1.737 | 0.205 | 0.278 | 0.398 | 0.338 |

**Table 4. influence of Nonionic Surfactants in the Ovalbumin Measurement System**

| Surfactant concentration (%) | Nonionic surfactant | | | Steroid surfactant |
|---|---|---|---|---|
| | Tween20 | LubrolPX | TritonX-100 | DOC |
| 0.005 | 1.769 | 1.859 | 1.667 | 1.888 |
| 0.014 | 1.713 | 1.803 | 1.837 | 1.889 |
| 0.041 | 1.718 | 1.727 | 1.847 | 1.895 |
| 0.123 | 1.786 | 1.710 | 1.828 | 1.820 |
| 0.370 | 1.754 | 1.609 | 1.788 | 1.830 |
| 1.110 | 1.743 | 1.658 | 1.812 | 1.627 |
| 3.330 | 1.780 | 1.612 | 1.791 | 1.383 |
| 10.000 | 1.668 | 1.399 | 1.735 | 1.199 |

**Table 5. Influence of Nonionic Surfactants in the Ovomucoid Measurement System**

| Surfactant concentration (%) | Nonionic surfactant | | | Steroid surfactant |
|---|---|---|---|---|
| | Tween20 | LubrolPX | TritonX-100 | DOC |
| 0.005 | 1.762 | 1.979 | 1.988 | 1.867 |
| 0.014 | 1.845 | 1.943 | 1.937 | 2.028 |
| 0.041 | 1.899 | 1.866 | 1.915 | 2.0223 |
| 0.123 | 1.832 | 1.877 | 1.894 | 1.940 |
| 0.370 | 1.879 | 1.846 | 1.869 | 1.866 |
| 1.110 | 1.836 | 1.877 | 1.900 | 1.775 |
| 3.330 | 1.844 | 1.780 | 1.869 | 1.733 |
| 10.000 | 1.778 | 1.117 | 1.645 | 1.447 |

From the results of this test, it was revealed that in the ovalbumin measurement system, the measurement sensitivity is significantly deteriorated and the immunoassay is inhibited by an ionic surfactant at relatively high concentration (for example 0.1% or more), while in the ovomucoid measurement system, the measurement is sufficiently feasible even in the presence of an ionic surfactant even at a very high concentration of about 10%. It was thus evidenced that even the anti-native protein antibody excluding the anti-(native) ovalbumin antibody can be used to provide an immunoassay system achieving excellent detection sensitivity and accuracy in combination with the high protein extraction ability of the ionic surfactant in the present invention. The nonionic surfactants and deoxycholic acid for reference did not exert influence in either the ovalbumin or ovomucoid measurement system.

### Example 3

### Measurement of casein and β-lactoglobulin (use of an anti-native-protein antibody)

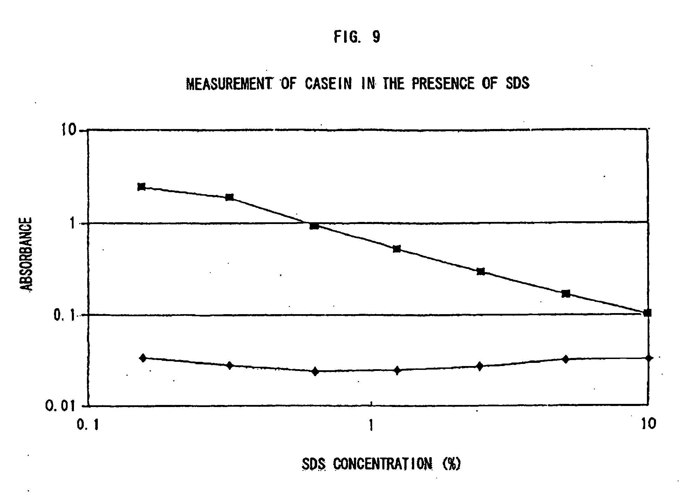

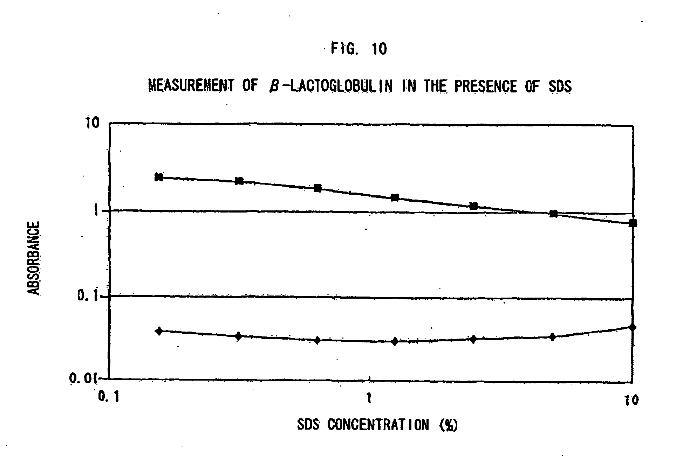

Casein or β-lactoglobulin was measured in almost the same procedure as in Example 1 except that an anti-(native) casein antibody or an anti-(native) P-lactoglobulin antibody was used in place of the anti-(native) ovomucoid antibody. That is, milk protein was added in an amount of 64 ng/ml to a sample diluent serially diluted to concentrations of 10% to 0.156% SDS, to give a sample solution for measurement. Casein and P-lactoglobulin in the sample were measured respectively by a commercially available milk immunoassay kit for casein and a milk immunoassay kit for β-lactoglobulin both using an antibody to native protein (trade name, Morinaga immunoassay kit for specific material, Milk measurement kit (casein); Morinaga immunoassay kit for specific material, Milk measurement kit (β-lactoglobulin)) available from Morinaga Institute of Biological Science Co., Ltd.,. The results are shown in Figs. 9 and 10.

From Figs. 9 and 10, it was revealed that even if the antibody to the native protein is used, the immunoassay of casein and β-lactoglobulin can also be carried out satisfactorily in the presence of the ionic surfactant SDS at high concentration.

### Example 4

### Measurement of buckwheat protein, wheat protein and peanut protein (use of an anti-native-protein antibody)

The influence of an ionic surfactant on measurement of buckwheat protein, wheat protein (gliadin) and peanut protein was verified in the same manner as in Example 3. The buckwheat protein used in the recovery experiment was prepared as follows: Buckwheat grains were milled and then extracted with a buffer solution (Tris-HCl buffer etc.) containing a salt such as sodium chloride, then the resulting extract was centrifuged to recover its supernatant, and the supernatant was applied to gel filtration columns Superdex® G-200 or Superose® 6 (both manufactured by Amersham Pharmacia), and fractions eluted in the range of molecular weights of 70 to 500 kD were recovered.

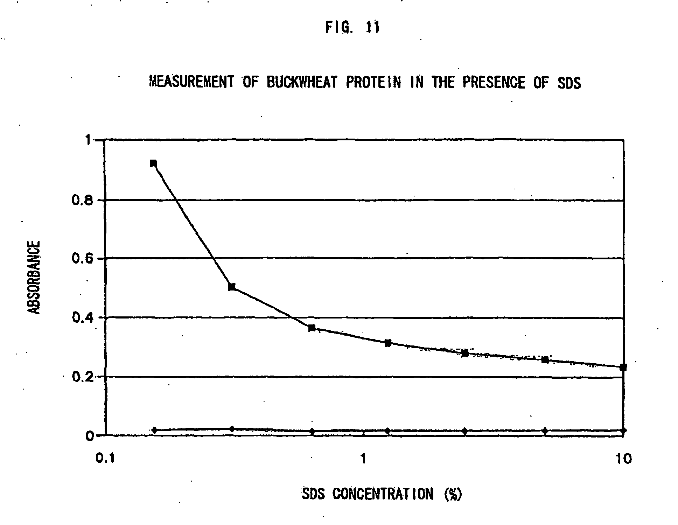

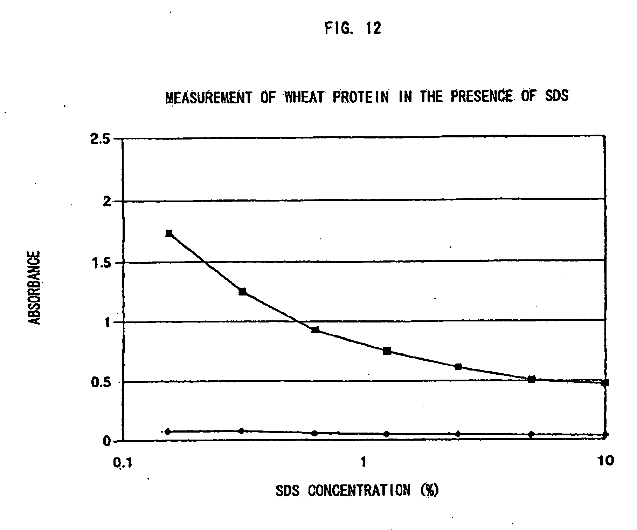

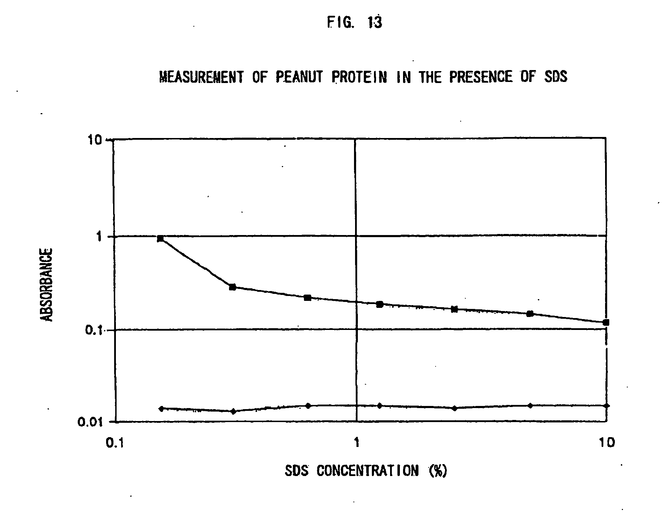

The wheat protein (gliadin) used was a commercially available product obtained from Asama Kasei Co., Ltd. The peanut protein was prepared as follows: Peanuts were milled and then extracted with a buffer solution (Tris-HCl buffer etc.) containing a salt such as sodium chlo-, ride, then the resulting extract was centrifuged to recover its supernatant, and the supernatant was applied to gel filtration columns Superdex® G-200 or Superose® 6 (both manufactured by Amersham Pharmacia), and fractions eluted in the range of molecular weights of 30 to 100 kD were recovered as the peanut protein. The immunoassay test using these samples was carried out by using commercially available immunoassay kits (Morinaga immunoassay kit for specific material, Buckwheat measurement kit; Morinaga immunoassay kit for specific material, Wheat measurement kit (gliadin); and Morinaga immunoassay kit for specific material, Peanut measurement kit, any of which use an antibody to native protein) available from Morinaga Institute of Biological Science Co., Ltd.,. The results are shown in Figs. 11 to 13.

From Figs. 11 to 13, it was revealed that the immunoassay of buckwheat protein, wheat protein and peanut protein can be carried out satisfactorily with the antibody to the native protein, in the presence of the ionic surfactant at high concentration.

### Example 5

### Preparation of an anti-ionic surfactant-denatured protein antibody

In Examples 1 to 4, it was revealed that the specific antigen-antibody reaction in the immunoassay of ovomucoid, casein, β-lactoglobulin, buckwheat protein, wheat protein and peanut protein can be achieved in the presence of the ionic surfactant at high concentration even if antibodies raised against the native proteins are used, but such antigen-antibody reaction cannot be achieved in the immunoassay of ovalbumin. Accordingly, a method of utilizing an antibody to a protein previously denatured by an ionic surfactant treatment was examined in the following manner.

### (1) Denaturation of proteins

The following proteins were dissolved at a concentration of 0.1 to 10 mg/ml in a solution containing 1% SDS and 1M 2-mercaptoethanol and then stood still overnight at room temperature.
1. Ovalbumin (trade name: Egg Albumin, 5x Cryst. (Chicken), purchased from Seikagaku Corporation)
2. Ovomucoid (trade name: Trypsin inhibitor (from chicken egg white), purchased from Nacalai Tesque)
3. Peanut protein was prepared in the following manner.
   1) Peanuts are milled and then extracted with a buffer solution (Tris-HCl buffer etc.) containing a salt such as sodium chloride.
   2) The extract is centrifuged to recover a supernatant.
   3) The supernatant is applied to gel filtration columns Superdex® G-200 or Superose® 6 (both manufactured by Amersham Pharmacia), and fractions eluted in the range of molecular weights of 30 to 100 kD are recovered.
4. Buckwheat protein was prepared in the following manner.
   1) Buckwheat grains are milled and then extracted with a buffer solution (Tris-HCl buffer etc.) containing a salt such as sodium chloride.
   2) The extract is centrifuged to recover a supernatant.
   3) The supernatant is applied to gel filtration columns Superdex® G-200 or Superose® 6 (both manufactured by Amersham Pharmacia), and fractions eluted in the range of molecular weights of 70 to 500 kD are recovered.

### (2) Preparation of rabbit polyclonal antibody

Using Freund adjuvant, each protein denatured as described above was emulsified, and the resultant emulsion was injected subcutaneously into a rabbit to be immunized. 1 mg of the denatured protein was administered for each immunization, and this administration was carried out 5 times at one-week intervals. 1 week after the final immunization, the whole blood of the immunized rabbit was collected to prepare an antiserum. Preparation of the antibody of the present invention from the antiserum was carried out according to the following procedure. That is, the denatured protein used in immunization of the rabbit was immobilized via a covalent bond onto resin HiTrap® NHS-activated (manufactured by Amersham Pharmacia), and the antiserum was applied to this immobilization resin. Then, the antibody bound to the protein fraction on the immobilization resin was eluted with 0.1M Gly-HCl adjusted to pH 2.7, to give the antibody of the present invention.

### Example 6

### Measurement by the anti-ionic surfactant-denatured protein antibody

The immunoassay (ELISA) with the antibody to the ionic surfactant-denatured protein according to the present invention was evaluated on the basis of the following protocol.

### [Immobilization of the antibody]

1. A solution of 1 µg/mL rabbit polyclonal antibody to the ionic surfactant-denatured protein as above in a carbonate buffer (pH 9.6) is prepared.
2. The solution was pipetted in a volume of 100 µL/well into a microtiter plate (Micromodule plate, Maxsorp-F8, manufactured by Nunc) and then left at ambient temperature for 2 hours.

### [Blocking]

1. After immobilization of the antibody, the antibody solution is removed, and 300 µL blocking solution (20 mM Tris-HCl buffer (pH 7.4) containing 150 mM NaCl, 0.05% Tween 20, 0.1% bovine serum albumin) is added to each well and left for 2 hours.

### [Preparation of a sample solution]

1. Standard protein is dissolved in a buffer solution (PBS, pH 6.5) containing 1% SDS and 1M 2-mercaptoethanol and then heated for 1 to 10 minutes by boiling in a hot water bath at 100°C.
2. After heating, the standard protein is diluted with the same buffer solution as above to concentrations of 1 to 64 ng/mL.

### [Measurement]

1. The solution of the standard protein at each concentration is added in a volume of 100 µL/well to the antibody-immobilized plate after blocking, and then subjected to stationary reaction for 1 hour at ambient temperature.
2. After the reaction, each well was washed 6 times with 300 µL washing solution, and a solution of horseradish peroxidase-labeled antibody in 20 mM Tris-HCl buffer (pH 7.4) containing 0.1% BSA, 150 mM NaCl and 0.05% Tween 20 was added in a volume of 100 pL/well, and then subjected to stationary reaction at ambient temperature for 30 minutes.
3. Each well was washed 6 times with 300 µL washing solution, and a solution of TMB (3,3',5,5'-tetramethylbenzidine) was added in a volume of 100 µL/well, and then reacted in the dark at ambient temperature for 10 minutes.
4. 100 µL of 1N sulfuric acid was added to each well to terminate the reaction. The absorbance of each well was measured at a major wavelength of 450 nm and a side wavelength of 630 nm with a microtiter plate reader. The results shown are average values in duplicate measurements.

### (1) Measurement of ovalbumin

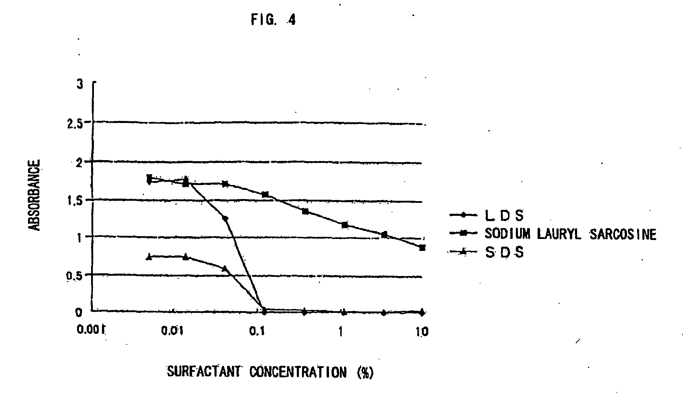

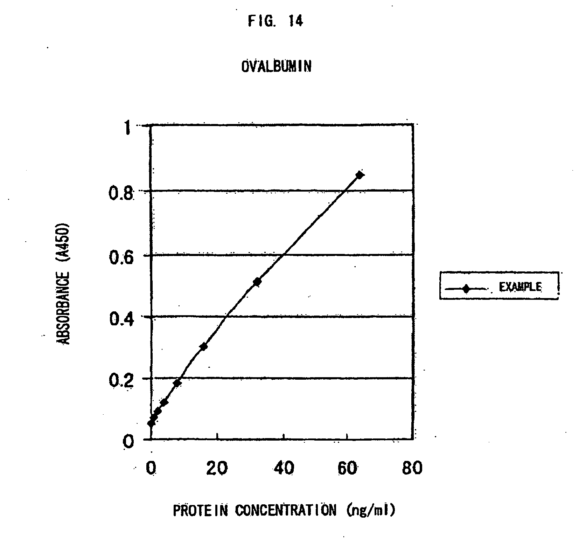

Ovalbumin which could not be measured with an antibody to the native protein (see Figs. 3 and 4) was measured by using the anti-ionic surfactant-denatured protein antibody according to the present invention. The measurement result is shown in Fig. 14. In this experiment, a solution of ovalbumin at each concentration in a buffer solution (PBS, pH 6.5) containing 1% SDS and 1M 2-mercaptoethanol was heated for 5 minutes in a hot water bath at 100°C, then cooled, and examined by ELISA described above.

As can be seen from Fig. 14, even the protein hardly measured with an antibody to the native protein in the presence of the ionic surfactant at high concentration can be measured with very high sensitivity by using the anti-ionic surfactant-denatured protein antibody.

### (2) Effect of boiling a sample solution

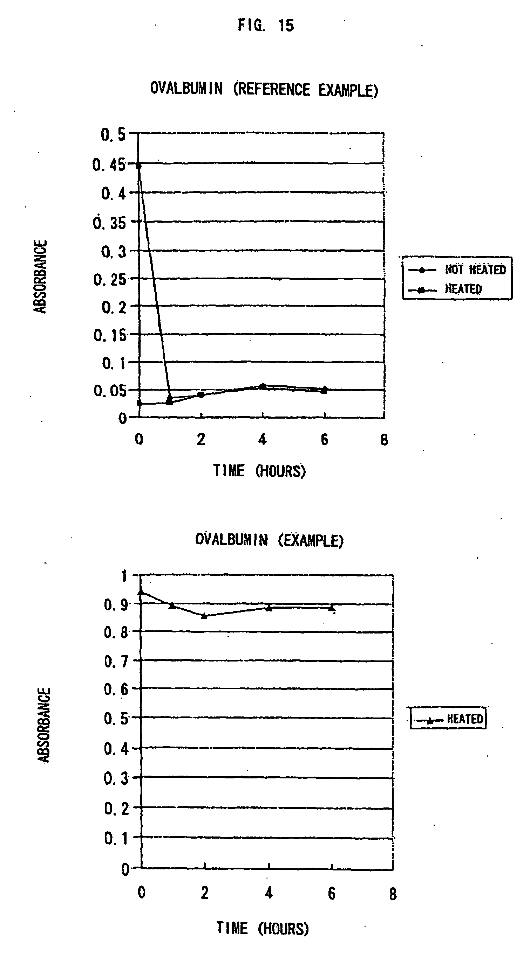

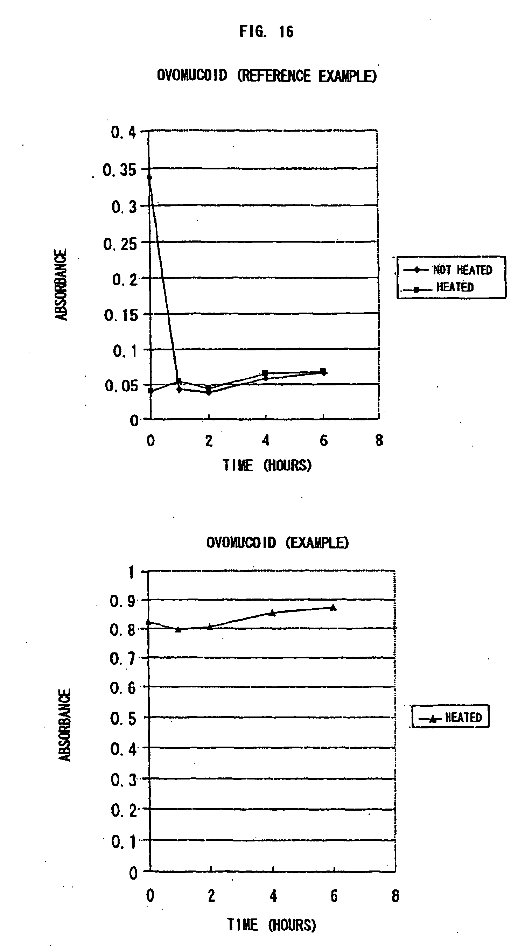

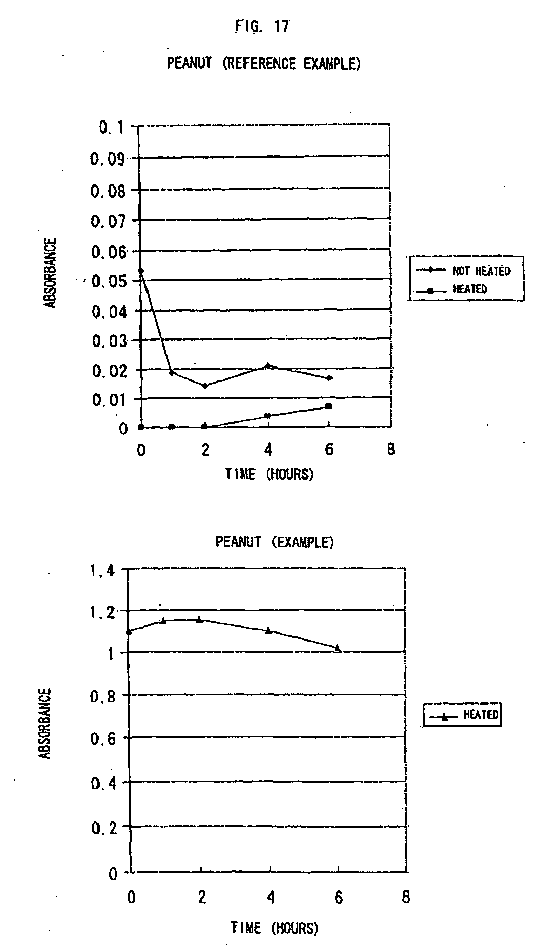

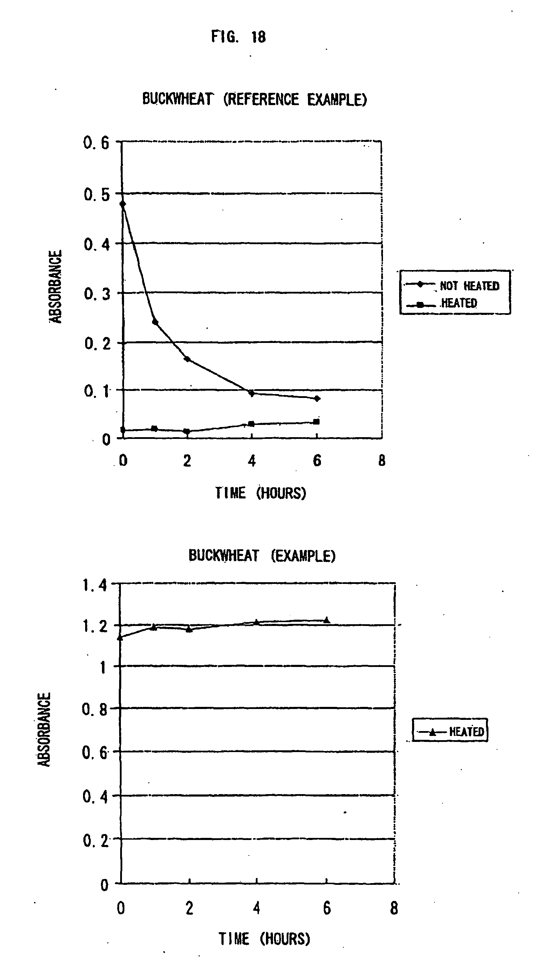

It was revealed that in the method of using the anti-ionic surfactant-denatured protein antibody according to the present invention, measurement stability and sensitivity can be significantly improved by previously boiling a sample solution. The results are shown in Figs. 15 to 18.

The example shows the result of measurement where each protein was dissolved at a concentration of 64 ng/ml in a buffer solution (PBS, pH 6.5) containing 1% SDS and 1M 2-mercaptoethanol to prepare a sample solution, and the sample solution was heated for 5 minutes in a hot water bath at 100°C, then left at room temperature for 0 to 6 hours and measured by ELISA with the anti-ionic surfactant-denatured protein antibody according to the present invention. On one hand, the reference example shows the result of measurement where an antibody to the native protein was used, and the sample solution was heated or not heated and then used in measurement. The result indicates that when the antibody to the native protein was used and the sample solution was not heated, high detection sensitivity could be maintained for a relatively short time, but the sensitivity was reduced with time. It was further revealed that when the antibody to the native protein was used and the sample solution was heated, detection sensitivity was significantly reduced from the start.

On the other hand, it was revealed that when the anti-ionic surfactant-denatured protein antibody of the present invention was used and the sample solution was previously boiled, high detection sensitivity was maintained for a long time, and the assay can be carried out with extremely high reproducibility and sensitivity.

### (3) Assay sensitivity

The sensitivity of the assay in a preferable aspect of the present invention where use of the anti-ionic surfactant-denatured protein antibody was combined with boiling of the sample solution is shown in Figs. 19 to 22. The example shows the result of measurement where each protein was dissolved at each concentration in a buffer solution (PBS, pH 6.5) containing 1% SDS and 1M 2-mercaptoethanol to prepare a sample solution, and the sample solution was heated for 5 minutes in a hot water bath at 100°C, then cooled and measured by ELISA. The reference example in the graph shows the result of measurement where the antibody to the native protein was used, and the sample solution was heated. As can be seen from the graphs, the method of the present invention has very high reproducibility and sensitivity.

### Industrial Applicability

By using the anti-ionic surfactant-denatured protein antibody of the present invention, the immunoassay of protein can be carried out satisfactorily even in the presence of an ionic surfactant at high concentration, as is evident particularly from Example 6. Accordingly, a sparingly soluble/hardly extractable protein which was solubilized/extracted by using the excellent effect of the ionic surfactant on solubilization of protein can be detected directly by the immunoassay, and the method of the present invention can be utilized very effectively in study on life science and in guaranteeing food qualities, requiring such high-sensitive detection.

## Claims

1. An immunoassay for detecting the presence in a sample of a protein that is water-sparingly-soluble and/or hardly extractable, i.e. a protein that shows at least a fivefold improved solubility and/or extraction efficiency in an aqueous solvent containing an ionic surfactant as compared to ionic surfactant-free pure water or a buffer solution, comprising the steps of:
(1) extracting and/or solubilizing said water-sparingly-soluble/hardly extractable protein in the sample with an aqueous solvent containing an ionic surfactant, wherein the concentration of the ionic surfactant in the aqueous solvent is higher than 0.3% (W/V),
(2) adding an antibody to the ionic surfactant-denatured protein obtained in step (1) to:
a) the protein solution obtained in the step (1) above without diluting the solution, or
b) a dilution of the protein solution obtained in the step (1) above,
whereby an antigen-antibody complex between the water-sparingly-soluble/hardly extractable protein and the antibody is formed, and
(3) detecting the formed antigen-antibody complex,
**characterized in that** the formation of the antigen-antibody complex in step (2) is carried out in the presence of the ionic surfactant at a concentration higher than 0.3% (W/V) and wherein the antibody in step 2 is an antibody previously raised against said ionic surfactant denatured protein.

2. The assay according to claim 1, wherein the ionic surfactant is selected from the group consisting of sodium dodecyl sulfate, lithium dodecyl sulfate, sodium lauryl sarcosine, hexadecyltrimethyl ammonium bromide, hexadecyltrimethyl ammonium chloride, hexadecyl pyridinium chloride and a mixture thereof.

3. The assay according to claim 2, wherein the ionic surfactant is sodium dodecyl sulfate.

4. The assay according to any one of claims 1 to 3, wherein the aqueous solvent in step (1) further comprises a reducing agent.

5. The assay according to claim 4, wherein the reducing agent is 2-mercaptoethanol, dithiothreitol or a mixture thereof.

6. The assay according to claim 5, wherein the aqueous solvent in step (1) comprises 1% (W/V) sodium dodecyl sulfate and 1M 2-mercaptoethanol.

7. The assay according to any one of claims 1 to 6, wherein in step (1), the protein solution is further boiled.

8. The assay according to claim 7, wherein the boiling is continued at least at 80°C for 5 minutes.

9. The assay according to any one of claims 1 to 8, wherein the protein is selected from the group consisting of ovalbumin, ovomucoid, casein, β-lactoglobulin, buckwheat protein, wheat protein and peanut protein which are in a hardly extractable state.

## Patentansprüche

1. Immuntest zum Erkennen der Gegenwart eines Proteins in einer Probe, welches schwer wasserlöslich und kaum extrahierbar ist, also eines Proteins, welches eine mindestens um das Fünffache verbesserte Löslichkeit und/oder Extraktionseffizienz in einem wässrigen Lösungsmittel aufweist, das eine ionische oberflächenaktive Substanz enthält, verglichen mit reinem Wasser, welches frei von ionischen oberflächenaktiven Substanzen ist, oder einer Pufferlösung, wobei der Immuntest die folgenden Schritte umfasst:
(1) Extrahieren und/oder Löslichmachen des schwer wasserlöslichen/kaum extrahierbaren Proteins in der Probe mit einem wässrigen Lösungsmittel, das eine ionische oberflächenaktive Substanz enthält, wobei die Konzentration der ionischen oberflächenaktiven Substanz in dem wässrigen Lösungsmittel höher als 0,3 % (w/v) ist,
(2) Zugebe eines Antikörpers zu dem durch die oberflächenaktive Substanz denaturierten Protein, das in Schritt (1) erhalten wird, zu:
a) der in Schritt (1) erhaltenen Proteinlösung ohne Verdünnen der Lösung oder
b) einer Verdünnung der in Schritt (1) erhaltenen Proteinlösung,
wodurch ein Antigen-Antikörper-Komplex zwischem dem schwer wasserlöslichen/kaum extrahierbaren Protein und dem Antikörper gebildet wird, und
(3) Erkennen des gebildeten Antigen-Antikörper-Komplexes, **dadurch gekennzeichnet, dass** die Bildung des Antigen-Antikörper-Komplexes in Schritt (2) in Gegenwart der ionischen oberflächenaktiven Substanz in einer Konzentration von mehr als 0,3 % (w/v) erfolgt, und wobei es sich bei dem Antikörper im Schritt (2) um einen Antikörper handelt, der zuvor gegen das durch die ionische oberflächenaktive Substanz denaturierte Protein erzeugt wurde.

2. Test nach Anspruch 1, wobei die ionische oberflächenaktive Substanz aus der Gruppe ausgewählt wird, die aus Natriumdodecylsulfat, Lithiumdodecylsulfat, Natriumlaurylsarkosin, Hexadecyltrimethylammoniumbromid, Hexadecyltrimethylammoniumchlorid, Hexadecylpyridiniumchlorid und einem Gemisch derselben besteht.

3. Test nach Anspruch 2, wobei es sich bei der ionischen oberflächenaktiven Substanz um Natriumdodecylsulfat handelt.

4. Test nach einem der Ansprüche 1 bis 3, wobei das wässrige Lösungsmittel in Schritt (1) ferner ein Reduktionsmittel umfasst.

5. Test nach Anspruch 4, wobei es sich bei dem Reduktionsmittel um 2-Mercaptoethanol, Dithiothreitol oder ein Gemisch derselben handelt.

6. Test nach Anspruch 5, wobei das wässrige Lösungsmittel in Schritt (1) 1 % (w/v) Natriumdodecylsulfat und 1 M 2-Mercaptoethanol umfasst.

7. Test nach einem der Ansprüche 1 bis 6, wobei in Schritt (1) ferner die Proteinlösung gekocht wird.

8. Test nach Anspruch 7, wobei 5 Minuten lang mindestens bei 80 °C gekocht wird.

9. Test nach einem der Ansprüche 1 bis 8, wobei das Protein aus der Gruppe ausgewählt wird, die aus Ovalbumin, Ovomucoid, Casein, β-Lactoglobulin, Buchweizenprotein, Weizenprotein und Erdnussprotein besteht, welche sich in einem kaum extrahierbaren Zustand befinden.

## Revendications

1. Dosage immunologique destiné à détecter la présence dans un échantillon d'une protéine faiblement soluble dans l'eau et/ou difficilement extractible, en d'autres termes une protéine qui présente une solubilité et/ou une efficacité d'extraction au moins 5 fois meilleure dans un solvant aqueux contenant un surfactant ionique comparé à une eau pure dépourvue de surfactant ionique ou à une solution tampon, comprenant les étapes suivantes :
(1) extraction et/ou solubilisation de ladite protéine faiblement soluble dans l'eau/difficilement extractible dans l'échantillon avec un solvant aqueux contenant un surfactant ionique, la concentration du surfactant ionique dans le solvant aqueux étant supérieure à 0,3 % (p/v),
(2) ajout d'un anticorps dirigé contre la protéine dénaturée par le surfactant ionique obtenue dans l'étape (1) à :
a) la solution protéique obtenue dans l'étape (1) ci-dessus sans diluer la solution, ou
b) une dilution de la solution protéique obtenue dans l'étape (1) ci-dessus,
un complexe antigène-anticorps entre la protéine faiblement soluble dans l'eau/difficilement extractible et l'anticorps étant ainsi formé, et
(3) détection du complexe antigène-anticorps formé, **caractérisé en ce que** la formation du complexe antigène-anticorps dans l'étape (2) est réalisée en présence du surfactant ionique à une concentration supérieure à 0,3 % (p/v) et l'anticorps dans l'étape 2 étant un anticorps préalablement développé pour être dirigé contre ladite protéine dénaturée par le surfactant ionique.

2. Dosage selon la revendication 1, le surfactant ionique étant choisi dans le groupe constitué par le dodécyl sulfate de sodium, le dodécyl sulfate de lithium, la lauryl sarcosine de sodium, le bromure d'hexadécyltriméthyl ammonium, le chlorure d'hexadécyltriméthyl ammonium, le chlorure d'hexadécyl pyridinium et un mélange de ceux-ci.

3. Dosage selon la revendication 2, le surfactant ionique étant le dodécyl sulfate de sodium.

4. Dosage selon l'une quelconque des revendications 1 à 3, le solvant aqueux dans l'étape (1) comprenant en outre un agent réducteur.

5. Dosage selon la revendication 4, l'agent réducteur étant le 2-mercaptoéthanol, le dithiothréitol ou un mélange de ceux-ci.

6. Dosage selon la revendication 5, le solvant aqueux dans l'étape (1) comprenant 1 % (p/v) de sodium dodécyl sulfate et du 2-mercaptoéthanol 1 M.

7. Dosage selon l'une quelconque des revendications 1 à 6, dans lequel dans l'étape (1), la solution protéique est en outre portée à ébullition.

8. Dosage selon la revendication 7, l'ébullition étant maintenue à au moins 80°C pendant 5 minutes.

9. Dosage selon l'une quelconque des revendications 1 à 8, la protéine étant choisie dans le groupe constitué par l'ovalbumine, l'ovomucoïde, la caséine, la β-lactoglobuline, la protéine de sarrasin, la protéine de blé et la protéine d'arachide qui se trouvent dans un état difificilement extractible.
